# EUROPEAN PATENT APPLICATION

(11) **EP 4 520 313 A1**
(43) Date of publication of application: **12.03.2025**
(21) Application number: 24190951.4
(22) Date of filing: 25.07.2024
(51) Int. Cl.: A61K 8/19, A61K 8/25, A61Q 11/00

(54) **COMPOSITION FOR DENTIFRICE**

(30) Priority: 31.07.2023 JP 2023124379
(71) Applicant: GC Corporation, Sunto-gun, Shizuoka 410-1307 (JP)
(72) Inventor: TOYONAGA, Kyohei, Tokyo, 174-8585 (JP); YASUI, Miyu, Tokyo, 174-8585 (JP); KATO, Shinichi, Tokyo, 174-8585 (JP)
(74) Representative: Müller-Boré & Partner Patentanwälte PartG mbB

(57) **Abstract**

The composition for dentifrice includes water; silica; and calcium chloride. Production of precipitates during storage of a composition for dentifrice that contains silica can be suppressed by said composition.

## Description

### FIELD

The present disclosure relates to a composition for dentifrice.

### BACKGROUND

It is known that dentifrice (compositions for dentifrice) is used in order to remove stains left on teeth which cause, for example, teeth shades. A composition for dentifrice may contain silica as a polishing agent. For example, silica is also an example of a polishing agent for the composition for dentifrice that is disclosed in patent literature 1.

### CITATION LIST

### Patent Literature

Patent Literature 1: JP 2006-69909 A

### SUMMARY

### Technical Problem

During storage of a conventional composition for dentifrice that contains silica as described above, precipitates may be produced on the surface of the composition.

An object of the present disclosure is to suppress, during storage of a composition for dentifrice that contains silica, production of precsipitates on the surface of the composition.

### Solution to Problem

As a result of their intensive studies, the inventors found that the precipitates produced on the surface during the storage was sodium sulfate, and thought in more detail as follows.

Sodium sulfate may be contained as an impurity while silica is being produced. Compositions for dentifrice also contain sodium sulfate as an impurity. Sodium sulfate does not precipitate immediately after a composition for dentifrice was stored because being disperse throughout the composition for dentifrice. However, sodium sulfate moves to the surface of the composition for dentifrice together with moisture in the process such that the moisture in the composition for dentifrice moves to the surface of the composition and vaporizes as time passes. Sodium sulfate remains on the surface because not evaporating. Therefore, sodium sulfate is concentrated and precipitates as crystals.

Then, the inventors conceived the following idea: it can be avoided that sodium sulfate moves together with moisture by converting sodium sulfate to a component insoluble in water before sodium sulfate moves to the surface of the composition for dentifrice, so that precipitation can be suppressed, and the inventors embodied this idea to complete the present invention.

The present application is a composition for dentifrice comprising: water; silica; and calcium chloride. Sodium fluoride may be further comprised.

A content of the calcium chloride in a content of the silica may be at least 0.01%.

### Advantageous Effects

According to the present disclosure, production of precipitates on the surface of a composition for dentifrice that contains silica, during storage of the composition, can be suppressed.

### DESCRIPTION OF EMBODIMENTS

The present disclosure will be described below with reference to embodiments thereof.

In the present embodiment, a composition for dentifrice is a pasty composition. The composition for dentifrice at least contains silica as a polishing agent (and a thickener), and further contains calcium chloride. Because of this, precipitates produced on the surface of the composition for dentifrice as described above can be reduced. Hereinafter, the composition for dentifrice will be described in more detail.

### [Water]

In this embodiment, the composition for dentifrice contains water that is preferably purified water. Water as used herein is preferably at least 5 mass%, and more preferably at least 15 mass% in an amount of the composition for dentifrice according to the present embodiment on one hand, and is preferably at most 30 mass%, and more preferably at most 25 mass% in an amount of the composition on the other hand. When the water is less than 5 mass% in an amount of the composition, dispersibility is poorer, which may make it difficult to lead to uniformity. Poor conditions are hardly created under the situation itself where the water is more than 30 mass% in an amount of the composition. However, the proportion of the other components becomes relatively low.

### [Moisturizer]

The composition for dentifrice according to the present embodiment can contain a moisturizer. As a moisturizer as used herein, any moisturizer conventionally used in compositions for dentifrice can be used. Specific examples of such a moisturizer include polyglycerol such as glycerol and diglycerol, propylene glycol, dipropylene glycol, sorbitol, xylitol, mannitol, ethylene glycol, diethylene glycol, polyethylene glycol, and monomethyl ether. Among them, two or more may be used in combination. Preferably, the composition for dentifrice according to the present embodiment contains at least one of glycerol, propylene glycol, and sorbitol.

The content of the moisturizer in the composition for dentifrice is appropriately adjusted according to materials to be used. The composition for dentifrice according to the present embodiment can contain 10 mass% to 60 mass%, preferably 20 mass% to 50 mass% of the moisturizer in an amount of the composition. When the content of the moisturizer is less than 10 mass% in an amount of the composition, the composition is susceptible to drying and may be incapable of holding a pasty form thereof. When the content of the moisturizer is more than 60 mass% in an amount of the composition, fluidity is improved, which may lead to bad conditions in use of the composition: for example, the composition is dripping from a brush.

### [Polishing Agent]

The composition for dentifrice according to the present embodiment contains a polishing agent. Silica shall be contained as a polishing agent as used herein. Any material other than silica is not prevented from being also contained as the polishing agent. Any polishing agent conventionally used for compositions for dentifrice may be used in combination. Examples of a polishing agent other than silica as used herein include aluminas, and mixtures thereof. An example of a commercially available silica as used herein is ZEODENT (registered trademark).

The ratio of combination of the polishing agent to the composition for dentifrice is appropriately adjusted according to materials to be used. The composition for dentifrice according to the present embodiment can contain 10 mass% to 40 mass%, preferably 20 mass% to 30 mass% of the polishing agent in an amount of the composition. When the content of the polishing agent is less than 10 mass% in an amount of the composition, cleaning power may be reduced. When the content of the polishing agent is more than 40 mass% in an amount of the composition, the pasty form cannot be maintained.

### [Precipitation Inhibitor]

In this embodiment, the composition for a dentifrice contains a precipitation inhibitor. Calcium chloride is used as a precipitation inhibitor as used herein. Because of this, production of precipitates on the surface of the composition for dentifrice during the storage of the composition can be suppressed.

In this embodiment, the composition for dentifrice contains silica for the polishing agent as described above. While silica is being produced, sodium sulfate may be contained as an impurity. In this case, the composition for dentifrice may also contain sodium sulfate as an impurity. Sodium sulfate in this case does not precipitate immediately after the start of the storage of the composition for dentifrice because being disperse throughout the composition. However, since being water-soluble, sodium sulfate moves to the surface of the composition for dentifrice together with moisture in the process such that the moisture in the composition for dentifrice moves to the surface of the composition and vaporizes as time passes. Then, sodium sulfate remains on the surface because the moisture vaporizes but sodium sulfate does not evaporate. Therefore, sodium sulfate is concentrated and precipitates as crystals.

In contrast, when the composition for dentifrice contains calcium chloride, sodium sulfate reacts with calcium chloride to form calcium sulfate. Since being insoluble in water, calcium sulfate hardly follows the movement of the moisture, and the precipitation is also suppressed.

The content of calcium chloride in the composition for dentifrice is not particularly limited as long as precipitation of sodium sulfate can be suppressed. This content is preferably 0.01% to 16%, more preferably 0.1% to 6%, and further preferably 1% to 4% of the silica content. When this content is less than 0.01%, the effect of suppressing the precipitation may not be capable of being sufficiently obtained. When this content is more than 6%, the composition tends to be more salty and bitter, but can be used because there is no problem in view of suppression in precipitation. Such a salty taste and bitterness are allowed as necessary, or can be adjusted by any other material to be milder.

### [Thickener]

The composition for dentifrice according to the present embodiment may contain a thickener in order to improve the viscosity of the composition. As a thickener as used herein, any thickener conventionally used for compositions for dentifrice can be used. Specific examples of such a thickener include polysaccharide thickeners such as sodium carboxymethylcellulose, sodium alginate, carboxy polymethylene, carboxymethyl cellulose, calcium carboxymethylcellulose, methyl vinyl ether-maleic anhydride copolymers, sodium starch glycolate, sodium starch phosphate, sodium polyacrylate, methyl cellulose, microcrystalline cellulose, hydroxypropyl cellulose, polyvinylpyrrolidone, vinyl polymers, xanthan gum, and carrageenan. Two or more of them may be used in combination.

The composition for dentifrice may contain an inorganic thickener. As an inorganic thickener as used herein, any inorganic thickener conventionally used for compositions for dentifrice can be used. Specific examples of such an inorganic thickener include calcium carbonate, calcium silicate, magnesium silicate, silicic acid powders, various glasses, amorphous hydrous silicic acid, fumed silica, titanium dioxide, and LiNaMg-silicate. Two or more of them may be used in combination. The inorganic thickener may be used in combination with any of the aforementioned polysaccharide thickeners. As described above, silica may be also contained in the thickener. However, usually, silica contained in the thickener does not contain sodium sulfate as an impurity.

The content of the thickener is appropriately adjusted according to materials to be used. The composition for dentifrice according to the present embodiment can contain 1 mass% to 10 mass%, preferably 2 mass% to 8 mass% of the thickener in an amount of the composition.

### [Others]

Other than the foregoing, in the composition for dentifrice according to the present embodiment, any of coloring agents, preservatives, sweeteners, flavors, and anticaries materials may be appropriately incorporated as needed in such an amount that the properties of the composition for dentifrice are not affected adversely, or are not substantially affected adversely.

Any coloring agent conventionally used for compositions for dentifrice may be used as a coloring agent as used herein. The coloring agent may be a water-soluble coloring agent. Specific examples of the coloring agent include titanium dioxide and zinc oxide. Titanium dioxide as used herein is a white powder that is to add opacity to the composition for dentifrice.

The content of the coloring agent is appropriately adjusted according to materials to be used. The composition for dentifrice according to the present embodiment can contain 0 mass% to 5 mass% of the coloring agent in an amount of the composition. A more preferred content of the coloring agent is 0.5 mass% to 2 mass% of the composition.

Any preservative conventionally used for compositions for dentifrice may be used as a preservative as used herein. Specific examples of the preservative include ethylparaben and propylparaben.

The content of the preservative is appropriately adjusted according to materials to be used. The composition for dentifrice according to the present embodiment can contain 0 mass% to 2 mass% of the preservative in percentage of the composition. A more preferred percentage of the preservative is 0.1 mass% to 0.5 mass% of the composition.

Any sweetener and/or flavor conventionally used for compositions for dentifrice may be used as a sweetener and/or flavor as used herein.

Specific examples of the sweetener include sucrose, sucralose, lactose, maltose, sorbitol, xylitol, sodium cyclamate, perillartine, AMP (aspartyl phenyl alanine, methyl ester), and sodium saccharine.

Specific examples of the flavor include: flavoring oils such as oils of spearmint, peppermint (menthol), wintergreen, sassafras, cloves, sage, eucalyptus, marjoram, cinnamon, lemons, and oranges; and methyl salicylate. Preferably, the composition for dentifrice according to the present embodiment contains methyl salicylate.

The content of the sweetener and/or flavor is appropriately adjusted according to materials to be used. The composition for dentifrice can contain 0 mass% to 10 mass% of the sweetener and/or flavor in percentage of the composition. Preferably, the sweetener and/or flavor is 0.5 mass% to 3 mass% in an amount of the composition.

In the composition for dentifrice according to the present embodiment, any material from which an anticaries effect can be expected, such as sodium fluoride, may be contained. It is sufficient to contain this material in such an amount that an anticaries effect can be expected. Preferably, this material is 0 mass% to 10 mass% of the composition. Because of features of the effect expected as a composition for dentifrice, at least some amount of sodium fluoride is preferably contained in an anticaries view. Specifically, preferably at least 1 mass%, more preferably at least 2 mass% of sodium fluoride is contained.

According to inventors' studies, a high content of sodium fluoride tends to cause precipitates that lead to some trouble to be more. In the present disclosure, the precipitates can be reduced even when much sodium fluoride is contained, and in such a view, the precipitates can be reduced as an anticaries function is maintained.

### Examples

The present disclosure will now be further described with reference to the following examples. The present disclosure is not limited to these examples.

### [Details of Composition]

In each of examples 1 to 7, a composition for dentifrice was prepared as shown in table 1. Example 1 corresponds to comparative example because calcium chloride was not contained therein. Examples 2 to 7 correspond to examples because different contents of calcium chloride were contained therein, respectively.

The compositions for dentifrice in the examples were each obtainable by mixing and stirring all the materials that were to constitute components thereof.

Table 1 also shows the percentage (%) of calcium chloride in silica.

### [Test Method and Evaluation]

For each of the examples, a plastic container (the top face thereof was an opening) was filled with the composition for dentifrice, and the opening of the container was closed with a sealing material, so that the container was sealed. The sealed container was put into a bag having an aluminum-deposited surface, and had been stored at 60°C for 2 weeks (accelerated test).

After the storage, the sealing material was removed, and the surface of the composition for dentifrice was visually observed to be evaluated based on the following criteria:
-: no precipitate was found;
±: slightly precipitated; and
+: precipitated.

The evaluation results are also shown in table 1.

**[Table 1]**

| (Table 1) | | | | | | | (unit: mass%) | |
|---|---|---|---|---|---|---|---|---|
| | | Example 1 | Example 2 | Example 3 | Example 4 | Example 5 | Example 6 | Example 7 |
| Moisturizer | glycerol | 15 | 15 | 15 | 15 | 15 | 15 | 15 |
| | propylene glycol | 30.369 | 30.369 | 30.369 | 30.369 | 30.369 | 30.369 | 30.369 |
| Polishing Agent | silica | 24 | 24 | 24 | 24 | 24 | 24 | 24 |
| Precipitation Inhibitor | calcium chloride dihydrate | 0 | 0.24 | 0.48 | 0.96 | 0.012 | 0.12 | 0.24 |
| Thickener | polyvinylpyrrolidone | 4 | 4 | 4 | 4 | 4 | 4 | 4 |
| | LiNaMg-silicate | 2 | 2 | 2 | 2 | 2 | 2 | 2 |
| Preservative | ethylparaben | 0.056 | 0.056 | 0.056 | 0.056 | 0.056 | 0.056 | 0.056 |
| | propylparaben | 0.026 | 0.026 | 0.026 | 0.026 | 0.026 | 0.026 | 0.026 |
| Sweetener | xylitol | 1 | 1 | 1 | 1 | 1 | 1 | 1 |
| Anticaries agent | sodium fluoride | 2.7 | 2.7 | 2.7 | 2.7 | 0.3 | 0.3 | 0.3 |
| Flavor | menthol | 0.149 | 0.149 | 0.149 | 0.149 | 0.149 | 0.149 | 0.149 |
| | other flavor(s) | 0.5 | 0.5 | 0.5 | 0.5 | 0.5 | 0.5 | 0.5 |
| Coloring agent | titanium oxide | 1 | 1 | 1 | 1 | 1 | 1 | 1 |
| Water | purified water | 19.2 | 18.96 | 18.72 | 18.24 | 21.588 | 21.48 | 21.36 |
| Total (mass%) | | 100 | 100 | 100 | 100 | 100 | 100 | 100 |
| Percentage of calcium chloride in silica (%) | | 0 | 1 | 2 | 4 | 0.05 | 0.5 | 1 |
| Results | | + | ± | - | - | - | - | - |

As the above, production of precipitates was capable of being suppressed by containing calcium chloride.

Among the above, particularly, production of precipitates was capable of being more reliably suppressed when the percentage of calcium chloride in silica was more than 1%.

As the above, when, for example, examples 2 and 7 were compared, it was found that the results of precipitates were changed by reducing sodium fluoride. On the contrary, sodium fluoride may be necessary for securing functions of compositions for dentifrice. Even when it is essential to contain at least some amount of sodium fluoride, production of precipitates can be suppressed by containing sodium chloride as in the present disclosure.

## Claims

1. A composition for dentifrice, comprising:
water;
silica; and
calcium chloride.

2. The composition for dentifrice according to claim 1, wherein a content of the calcium chloride in a content of the silica is at least 0.01%.

3. The composition for dentifrice according to claim 1 or 2, the composition further comprising sodium fluoride.
